# EUROPEAN PATENT APPLICATION

(11) **EP 4 775 169 A1**
(43) Date of publication of application: **15.07.2026**
(21) Application number: 26151496.2
(22) Date of filing: 13.01.2026
(51) Int. Cl.: A61B 34/30, A61M 25/01, A61M 25/09, A61M 39/06

(54) **GEAR ASSEMBLY FOR HEMOSTASIS VALVE**

(30) Priority: 14.01.2025 US 202519020299
(71) Applicant: Siemens Healthineers Endovascular Robotics, Inc., Newton, MA 02466 (US)
(72) Inventor: BOUCHER, Wayne, Manchester, NH 03109 (US); FALB, Peter, Newton, MA 02466 (US)
(74) Representative: HKW Intellectual Property PartG mbB

(57) **Abstract**

A device includes an exterior surface comprising a driven element to interface with a drive element of a robotic drive, and an interior surface comprising features to fixedly couple to a rotator of a rotating hemostasis valve such that driving the driven element with the drive element causes movement of the rotator.

## Description

### BACKGROUND

Vascular disease may be treated in a variety of ways. For example, cardiac bypass surgery may be used to treat cardiovascular disease. Certain vascular diseases may be treated with catheter-based interventional procedures, such as angioplasty. Catheter-based interventions are generally considered safer and less invasive than surgery.

Catheter-based interventional procedures typically involve navigating a guidewire through patient vasculature and advancing a catheter, valve, stent, etc. via the guidewire to deliver therapy. According to one type of catheter-based interventional procedure, a guide catheter is inserted into a patient's femoral artery and positioned proximate the coronary ostium of a patient's heart. The guide catheter is connected to a distal end of a hemostasis valve. A guidewire is inserted through a proximal end of the hemostasis valve and into the guide catheter and is then maneuvered through the patient's arterial system until it reaches the site of a lesion. A working catheter comprising, for example, a balloon and a stent is moved along the guidewire until the working catheter is positioned proximate the lesion. The working catheter is deployed/activated to increase blood flow proximate the lesion.

Robotic catheter systems provide robotic manipulation of catheters, guidewires and other elongated medical devices (EMDs) during catheter-based interventional procedures. Generally, one or more elements are coupled to an EMD and the one or more elements are driven by one or more drive elements of a robotic drive in order to impart desired motion to the EMD. The movement may consist of rotation, linear translation, and/or any other type of movement.

A rotating hemostasis valve may include a rotating luer connector to which a catheter or other EMD is typically attached. In one example, a gear (i.e., a driven element) is attached to the rotating luer connector of the hemostasis valve and a second luer connector is coupled to the gear in-line with the rotating luer connector. A catheter is attached to the second luer connector and the robotic drive operates its drive elements to drive the gear in order to rotate the catheter.

Adding such a gear and second luer connector in-line with the hemostasis valve reduces the usable, or working, length of any EMDs (such as a guidewire) which pass through the hemostasis valve during an interventional procedure. The loss of working length for a given EMD is additive based on the number of hemostasis valves through which the EMD passes. Certain interventional procedures use up to three hemostasis valves in series, resulting in a lost working length of the most proximal EMD of at least three times the longitudinal length of the gear. The reduced working length results in a reduced capability of reaching distal targets during the procedure. Moreover, since the gear is a separate component from the hemostasis valve, the gear is at risk of separating from the hemostasis valve in high-torque scenarios.

Some rotating hemostasis valve include a rotating luer connector into which a gear is integrated. The gear and the rotating luer connector are a single inseparable unit. Although such an arrangement may prevent detachment of the gear from the valve and may avoid increasing the overall length of the rotating hemostasis valve, such rotating hemostasis valves require special tooling and manufacturing techniques. Moreover, such rotating hemostasis valves are compatible only with robotic drives having drive elements which correctly mate with the integrated gear.

Systems are desired to facilitate robotic control of commonly-available rotating hemostasis valves while preserving EMD working length.

According to a first aspect, a device is suggested, the device comprising an exterior surface comprising a driven element to interface with a drive element of a robotic drive, and an interior surface comprising features to fixedly couple to a rotator of a rotating hemostasis valve such that driving the driven element with the drive element causes motion of the rotator.

According to an embodiment, the motion comprises rotation of the rotator.

According to a further embodiment, the motion comprises longitudinal movement of the rotator.

According to a further embodiment, the motion comprises longitudinal movement of the rotator.

According to a further embodiment, the device does not extend beyond an end of a male luer connector of the rotating hemostasis valve when the features of the interior surface are fixedly coupled to the rotator.

According to a further embodiment, the device comprises a first portion and a second portion, the first portion and the second portion comprising one or more features to fixedly couple the first portion and the second portion when the features of the interior surface are fixedly coupled to the rotator.

According to a further embodiment, the first portion comprises the driven element and a first surface to interface with a first end of the rotator.

According to a further embodiment, the first portion comprises a second surface to interface with an end of a protrusion extending from a surface of the rotator.

According to a further embodiment, the first portion comprises a projection extending from the interior surface, and the second portion comprises a flexible portion defining an opening in which the projection fixedly resides when the features of the interior surface are fixedly coupled to the rotator.

According to a further embodiment, the projection biases the flexible portion to flex away from the projection as the first portion is slid over the second portion.

According to a further embodiment, the first portion comprises the features to fixedly couple to the rotator.

According to a further embodiment, the driven element comprises a gear to rotate the rotator and a feature to interface with the robotic drive to bias the device longitudinally.

According to a further embodiment, the device comprises a first surface to interface with a first end of the rotator and a second surface to interface with an end of a protrusion extending from a surface of the rotator.

According to a second aspect, a device is suggested, the device comprising an exterior surface comprising a driven element to interface with a drive element of a robotic drive, and an interior surface comprising first features to couple to second features of a rotator of a rotating hemostasis valve such that driving the driven element with the drive element causes movement of the rotator, wherein the device does not extend beyond an end of a male luer connector of the rotating hemostasis valve when the first features of the interior surface are coupled to the rotator.

According to a further embodiment, the device comprises a first portion and a second portion, each of the first portion and the second portion comprising one or more features to fixedly couple the first portion and the second portion when the first features of the interior surface are coupled to the second features of the rotator.

According to a further embodiment, the first portion comprises the driven element and a first surface to interface with a first end of the rotator, wherein the first portion comprises a second surface to interface with an end of one of the second features of the rotator.

According to a further embodiment, the first portion comprises a projection extending from the interior surface, wherein the second portion comprises a flexible portion defining an opening in which the projection fixedly resides when the first features of the interior surface are coupled to the second features of the rotator.

According to a further embodiment, the projection biases the flexible portion to flex away from the projection as the first portion is slid over the second portion.

According to a third aspect, a method is suggested, comprising: coupling a first portion of a device to a rotator of a rotating hemostasis valve; coupling a second portion of the device to the first portion and to the rotator of the rotating hemostasis valve; and inserting the rotating hemostasis valve into a robotic drive to couple a driven element of the second portion to a drive element of the robotic drive; and operating the drive element to drive the driven element, wherein the driving of the driven element causes movement of the rotator.

According to a further embodiment, coupling the second portion of the device to the first portion comprises sliding the second portion over the first portion to bias a projection extending from the second portion against a flexible portion of the first portion until the projection fixedly resides within an opening of the flexible portion.

### BRIEF DESCRIPTION OF THE DRAWINGS

Embodiments will become more fully understood from the following detailed description, taken in conjunction with the accompanying drawings, wherein the reference numerals refer to like parts in which:
FIG. 1 is a perspective view of a catheter-based procedure system in accordance with some embodiments;
FIG. 2 is a schematic block diagram of a catheter-based procedure system in accordance with some embodiments;
FIG. 3 is a perspective view of a robotic drive in accordance with an embodiment;
FIGS. 4A-4C are perspective views of an off-the-shelf rotating hemostasis valve according to some embodiments;
FIG. 5 illustrates attachment of a first portion of a device to a rotator of a rotating hemostasis valve according to some embodiments;
FIG. 6 illustrates attachment of a second portion of a device to a rotator of a rotating hemostasis valve according to some embodiments;
FIGS. 7A-7E are perspective views of a device attached to a rotator of a rotating hemostasis valve according to some embodiments;
FIGS. 8A-8D are perspective views of a second portion of a device including a driven element and a biasing projection according to some embodiments;
FIGS. 9A-9D are perspective views of a first portion of a device including a flexible element defining an opening according to some embodiments;
FIG. 10 is a cross-sectional view of a rotating hemostasis valve during coupling of a device thereto according to some embodiments;
FIG. 11 is a cross-sectional view of a rotating hemostasis valve during coupling of a device thereto according to some embodiments;
FIG. 12 is a cross-sectional view of a rotating hemostasis valve and a device coupled thereto according to some embodiments;
FIG. 13 is a perspective cross-sectional view of a rotating hemostasis valve and a device coupled thereto according to some embodiments;
FIG. 14 is a cross-sectional view of a rotating hemostasis valve and a device coupled thereto according to some embodiments;
FIG. 15 is a cross-sectional view of a rotating hemostasis valve and a device coupled thereto according to some embodiments;
FIG. 16 is a perspective view of a rotating hemostasis valve prior to insertion into a cassette of a robotic drive according to some embodiments;
FIG. 17 is a perspective view of a rotating hemostasis valve inserted into a cassette of a robotic drive according to some embodiments;
FIG. 18 is a perspective view of a cassette of a robotic drive after insertion of a rotating hemostasis valve according to some embodiments;
FIG. 19 is a side view of a rotating hemostasis valve and a device coupled thereto according to some embodiments;
FIG. 20 is a side view of a prior art system;
FIG. 21 is a side view of a robotic drive including cassettes supporting rotating hemostasis valves according to some embodiments; and
FIG. 22 is a side view of a robotic drive including cassettes supporting prior art rotating hemostasis valves.

### DETAILED DESCRIPTION

The following description is provided to enable any person in the art to make and use the described embodiments. Various modifications, however, will be readily-apparent to those in the art.

Some embodiments include a device having an exterior surface comprising a driven element to interface with a drive element of a robotic drive and an interior surface comprising features to fixedly couple to protrusions of a rotator of a rotating hemostasis valve such that driving the driven element with the drive element causes rotation of the rotating hemostasis valve. Advantageously, some embodiments enable the rotating hemostasis valve to be used by a robotic drive without increasing the effective length of the rotating hemostasis valve.

In some embodiments, the features of the interior surface of the device are fixedly coupled to the protrusions of the rotator of a rotating hemostasis valve. Such features may enable torque transmission from the driven element of the device to the rotator. The features may also tolerate higher torques compared to prior art systems in which the connections between the luer and the gear and between the hemostasis valve and the gear are susceptible to separation.

Some embodiments avoid the need for special tooling and/or manufacturing techniques to couple a driven element to a rotating hemostasis valve for use with a robotic drive. In one non-exhaustive example, the device comprises a first portion and a second portion, and the portions each include one or more features to fixedly couple the portions to one another when the features of the interior surface are fixedly coupled to the protrusions of the rotator.

As used herein, the term EMD refers to, but is not limited to, catheters (e.g., guide catheters, microcatheters, balloon/stent catheters), wire-based devices (e.g., guidewires, microwires, proximal pushers for embolization coils, stent retrievers, self-expanding stents, flow divertors, etc.), and medical devices comprising any combination of these.

The terms distal and proximal define relative locations of two different features. With respect to a robotic drive, the terms distal and proximal are defined by the position of the robotic drive in its intended use relative to a patient. When used to define a relative position, the distal feature is the feature of the robotic drive that is closer to the patient than a proximal feature when the robotic drive is in its intended in-use position. Within a patient, any vasculature landmark further away along the path from the access point is considered more distal than a landmark closer to the access point, where the access point is the point at which the EMD enters the patient. Similarly, the proximal feature is the feature that is farther from the patient than the distal feature when the robotic drive in its intended in-use position. When used to define direction, the distal direction refers to a path on which something is moving or is aimed to move or along which something is pointing or facing from a proximal feature toward a distal feature and/or patient when the robotic drive is in its intended in-use position. The proximal direction is the opposite direction of the distal direction.

The term longitudinal axis is the direction of orientation between a proximal portion and a distal portion. Axial movement refers to translation along a longitudinal axis. Rotational movement refers to clockwise or counterclockwise rotation about a longitudinal axis.

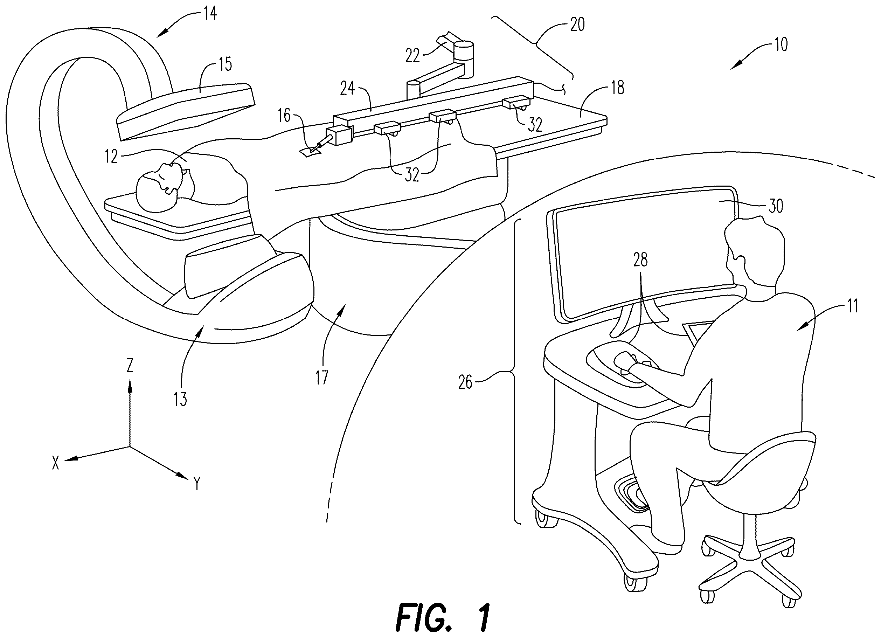

FIG. 1 is a perspective view of catheter-based procedure system 10 in accordance with some embodiments. Catheter-based procedure system 10 may be used to perform catheter-based procedures, e.g., percutaneous intervention procedures such as a percutaneous coronary intervention (e.g., to treat STEMI), a neurovascular intervention (e.g., to treat an emergent large vessel occlusion (ELVO)), peripheral vascular interventions (e.g., for critical limb ischemia (CLI), etc.). Catheter-based procedures may include diagnostic catheterization procedures during which one or more catheters or other EMDs are used to aid in the diagnosis of a patient's disease. In some procedures, a contrast media is injected into one or more arteries through a catheter and an image of the patient's vasculature is acquired while the contrast media resides therein.

Catheter-based procedures may also include catheter-based therapeutic procedures (e.g., angioplasty, stent placement, treatment of peripheral vascular disease, clot removal, arterial venous malformation therapy, treatment of aneurysm, etc.) during which a catheter (or other EMD) is used to treat a disease. Therapeutic procedures may be enhanced by the inclusion of adjunct devices such as, for example, intravascular ultrasound (IVUS), optical coherence tomography (OCT), and fractional flow reserve (FFR). Certain specific percutaneous intervention devices or components (e.g., type of guidewire, type of catheter may be selected based on the type of procedure that is to be performed. Catheter-based procedure system 10 can perform any number of catheter-based medical procedures with minor adjustments to accommodate the specific percutaneous intervention devices to be used in the procedures.

Catheter-based procedure system 10 includes, among other elements, bedside unit 20 and control station 26. Bedside unit 20 includes robotic drive 24 and positioning system 22 that are located adjacent to a patient table 18. Patient table 18 supports patient 12.

Positioning system 22 is used to position and support the robotic drive 24. Positioning system 22 may include, for example, a robotic arm, an articulated arm, and a holder. Positioning system 22 may be attached at one end to, for example, a rail of patient table 18, a base, or a cart. The other end of positioning system 22 is attached to robotic drive 24. Positioning system 22 may be moved (along with the robotic drive 24) to allow patient 12 to be placed on patient table 18. Once patient 12 is positioned on patient table 18, positioning system 22 may be used to situate or position robotic drive 24 relative to patient 12 for the procedure. In some embodiments, patient table 18 is operably supported by pedestal 17, which is secured to the floor and/or earth. Patient table 18 is able to move with multiple degrees of freedom, for example, roll, pitch, and yaw, relative to pedestal 17.

Bedside unit 20 may also include controls and displays as will be described below. For example, controls and displays may be located on the housing of robotic drive 24. Bedside unit 20, and in particular robotic drive 24, may include any number and/or combination of components to provide bedside unit 20 with the functionality described herein.

Generally, robotic drive 24 may be loaded with the percutaneous interventional devices and accessories appropriate for a given procedure. Examples of percutaneous interventional devices include but are not limited to guidewires, catheters, microwires, microcatheters, therapeutic devices including embolic coils, stents, flow divertors, and stent retrievers. Robotic drive 24 is elongated along the longitudinal axis of the patient table 18.

Robotic drive 24 is configured to move device modules 32, which may comprise a drive module and a cassette. Device modules 32 may be arranged in a vertical or horizontal configuration with respect to the patient table 18. Device modules 32 are configured to house and move percutaneous devices, including hemostasis valves. In one embodiment, cassettes are configured to house and drive percutaneous devices including hemostasis valves. The percutaneous devices may comprise adapters for interfacing with cassette elements as described herein.

Operator 11 controls robotic drive 24 to perform the procedure by operating various controls of control station 26. Bedside unit 20 is in communication with control station 26, allowing signals generated by the controls of control station 26 to be transmitted wirelessly or via wire to bedside unit 20 to control various functions of bedside unit 20, including functions of robotic drive 24. Bedside unit 20 may also provide feedback signals (e.g., loads, speeds, operating conditions, warning signals, error codes, etc.) to control station 26, the control computing system, or both.

Control station 26 may include a control computing system or be coupled to bedside unit 20 through a control computing system. Control station 26 includes input module 28 including controls configured according to some embodiments to receive user manipulations for controlling robotic drive 24 and/or various other components or systems of catheter-based procedure system 10. In the embodiment shown, control station 26 allows operator 11 to control bedside unit 20 to perform a catheter-based medical procedure. Robotic drive 24 includes various drive mechanisms to cause movement (e.g., linear and rotational movement) of the components of the bedside unit 20 including the percutaneous intervention devices in response to user manipulation of the controls of input module 28. For example, input module 28 may be configured to cause bedside unit 20 to perform various tasks using percutaneous intervention devices loaded into robotic drive 24. These tasks may include tasks to advance, retract, or rotate a guidewire, advance, retract or rotate a catheter, inflate or deflate a balloon located on a catheter, position and/or deploy a stent, position and/or deploy a stent retriever, position and/or deploy a coil, inject contrast media into a catheter, inject liquid embolics into a catheter, inject medicine or saline into a catheter, aspirate on a catheter, or to perform any other function that may be performed as part of a catheter-based medical procedure.

Input module 28 may include device selection buttons to allow operator 11 to select which of the percutaneous intervention devices loaded into the robotic drive 24 are controlled via user manipulation of input controls. Automated move buttons may be used to enable algorithmic movements that catheter-based procedure system 10 may perform on a percutaneous intervention device without direct command from operator 11.

Input module 28 may also include a balloon or stent control that is configured to instruct inflation or deflation of a balloon and/or deployment of a stent. Input module 28 may include one or more buttons, scroll wheels, joysticks, touch screen, etc. that is dedicated to instruct control of a particular component or components. In addition, one or more touch screens may display one or more icons (not shown) presenting relative positions of input modules 28 or various components of catheter-based procedure system 10. Such one or more touch screens may present a user interface for specifying and/or presenting a configuration of the controls of input module 28 and one or more functions, including but not limited to linear and/or rotational locking functions.

Control station 26 may include display 30. In some embodiments, control station 26 may include two or more displays 30. Display 30 may be configured to display information or patient specific data to operator 11 located at control station 26. For example, display 30 may be configured to display image data (e.g., X-ray images, MRI images, CT images, ultrasound images), hemodynamic data (e.g., blood pressure, heart rate), patient record information (e.g., medical history, age, weight), and lesion or treatment assessment data (e.g., IVUS, OCT, FFR). In addition, display 30 may be configured to display procedure specific information (e.g., procedural checklist, recommendations, duration of procedure, catheter or guidewire position, volume of medicine or contrast agent delivered. Display 30 may include touch screen capabilities to provide some of the user input capabilities of the system.

Catheter-based procedure system 10 also includes imaging system 14. Imaging system 14 may be any medical imaging system that may be used in conjunction with a catheter based medical procedure (e.g., non-digital X-ray, digital X-ray, CT, MRI, ultrasound). In an embodiment, imaging system 14 is a digital X-ray imaging device that is in communication with control station 26. In one embodiment, imaging system 14 may include a C-arm that allows imaging system 14 to partially or completely rotate around patient 12 in order to obtain images at different angular positions relative to patient 12 (e.g., sagittal views, caudal views, anterior-posterior views). Imaging system 14 may comprise a fluoroscopy system including a C-arm having X-ray source 13 and detector 15, also known as an image intensifier.

Imaging system 14 may be configured to acquire X-ray images of an appropriate area of patient 12 during a procedure. For example, imaging system 14 may be configured to acquire one or more X-ray images of the head to diagnose a neurovascular condition. Imaging system 14 may also be configured to take one or more X-ray images (e.g., real time images) during a catheter-based medical procedure to assist operator 11 of control station 26 to properly position a guidewire, guide catheter, microcatheter, stent retriever, coil, stent, balloon, etc. during the procedure. The image or images may be displayed on display 30. For example, images may be displayed on display 30 to allow operator 11 to accurately move a guide catheter or guidewire into the proper position.

In some embodiments, operator 11 and control station 26 are located in the same room as or an adjacent room to patient 12 and bedside unit 20. In other embodiments, control station 26 and bedside unit 20 are located away from one another, for example, in different rooms in the same building, different buildings in the same city, different cities, or other different locations where an operator located at control station 26 does not have convenient physical access to bedside unit 20 and/or patient 12. In the latter embodiments, operator 11 may communicate with personnel located proximate to bedside unit 20 to perform any physical manipulation of robotic drive 24 and/or EMDs which may be required during a procedure.

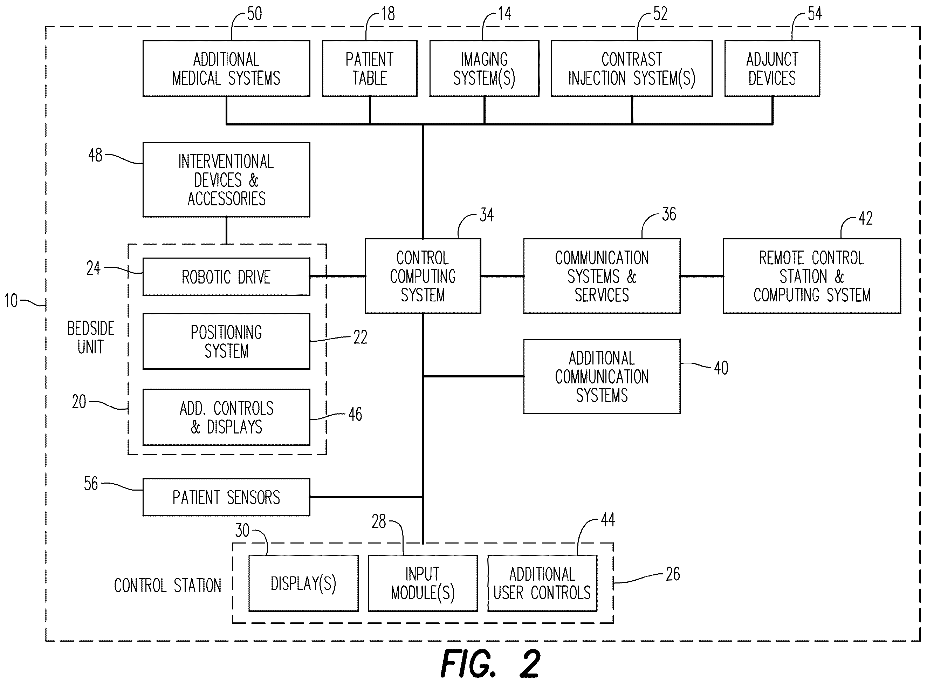

FIG. 2 is a block diagram of catheter-based procedure system 10 according to some embodiments. Catheter-procedure system 10 of FIG. 2 includes control computing system 34. In various embodiments, control computing system 34 is configured to receive and generate control signals based on user manipulation of the controls of input module 28 of control station 26, and/or based on information accessible to control computing system 34, such that a medical procedure may be performed using catheter-based procedure system 10.

Control computing system 34 may generally comprise a computer processing unit suitable to provide catheter-based procedure system 10 with the various functionalities described herein. For example, control computing system 34 may be an embedded system, a dedicated circuit, a general-purpose system programmed with the functionality described herein, etc. Control computing system 34 may, for example, be a component of control station 26 of FIG. 1.

Control computing system 34 is in communication with bedside unit 20, control station 26, additional communications systems 40 (e.g., a telepresence system), and patient sensors 56 (e.g., electrocardiogram (ECG) devices, electroencephalogram (EEG) devices, blood pressure monitors, temperature monitors, heart rate monitors, respiratory monitors). Control computing system 34 is also in communication with imaging system 14, patient table 18, additional medical systems 50, contrast injection systems 52 and adjunct devices 54 (e.g., IVUS, OCT, FFR. Bedside unit 20 includes robotic drive 24 and positioning system 22 as described above and may include additional controls and displays 46. Additional controls and displays 46 may be located on a housing of robotic drive 24.

Interventional devices and accessories 48 (e.g., guidewires, catheters) interface with bedside system 20. In some embodiments, interventional devices and accessories 48 may include specialized devices (e.g., EMDs including an internal member and an external member as described herein, IVUS catheter, OCT catheter, FFR wire, diagnostic catheter for contrast) which interface to their respective adjunct devices 54, namely, an IVUS system, an OCT system, and FFR system, etc.

Catheter-based procedure system 10 may be connected or configured to include any other systems and/or devices not explicitly shown. For example, catheter-based procedure system 10 may include image processing engines, data storage and archive systems, automatic balloon and/or stent inflation systems, medicine injection systems, medicine tracking and/or logging systems, user logs, encryption systems, systems to restrict access or use of catheter-based procedure system 10, etc.

As used herein, the term cassette generally refers to a component of a robotic drive including elements to support and move (e.g., rotate and/or translate) at least one EMD. A drive module generally refers to a component of a robotic drive that includes one or more motors with drive couplers which interface with the EMD-moving elements of a cassette. A cassette may provide a sterile interface between at least one EMD and a drive module directly or through a device adapter.

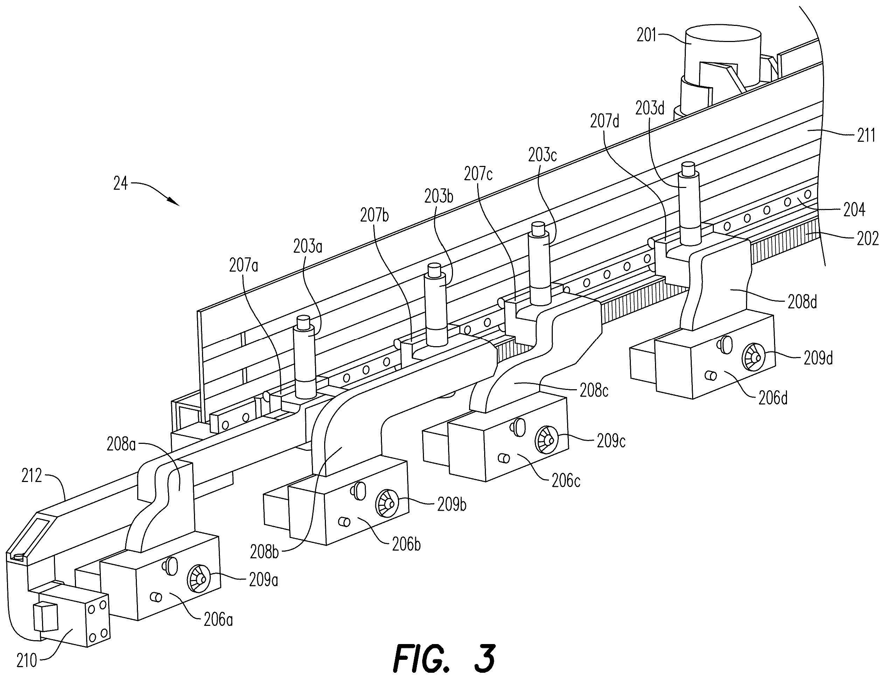

FIG. 3 is a perspective view of robotic drive 24 in accordance with some embodiments. Embodiments are not limited to robotic drive 24 of FIG. 3. Robotic drive 24 includes multiple drive modules 206a-d coupled to linear member 211. As will be discussed below, a respective cassette (not shown) may be mounted to each drive module 206a-d. In one embodiment, a cassette coupled to a drive module is referred to as a device module. Each cassette may be configured to house and drive a rotating hemostasis valve 100 with or without an adapter or device. During a procedure, drive modules 206a-d of FIG. 3 are in a vertical configuration with respect to a patient bed. The vertical orientation reduces the distance between robotic drive 24 and the patient and the distance between a longitudinal device axis of robotic drive 24 and an introducer sheath with respect to other drive module configurations, thereby reducing a loss of working length of all EMDs loaded into drive modules 206a-d.

During a procedure, an EMD may be loaded into one or more of the cassettes. Each cassette may include elements to move an EMD loaded therein in one or more degrees of freedom. Each drive module 206a-d includes at least one coupler 209a-d to interface with such elements in each cassette. Each drive module 206a-d also includes a motor (not shown) that is used to rotate its corresponding coupler 209a-d. Accordingly, rotation of a coupler 209a-d by a motor of its corresponding drive module 206a-d may cause the coupler 209a-d to drive mechanisms in the cassette mounted thereto to cause, for example, rotation of an EMD loaded in the cassette.

Each drive module 206a-d is coupled to linear member 211 via stage (or slide) 203a-d moveably mounted to rail 204 of linear member 211. A drive module 206a-d may be connected to its stage 203a-d using a connector such as an offset bracket 208a-d. In another embodiment, a drive module 206a-d may be directly mounted to its stage 203a-d.

Robotic drive 24 may also include device support connection 210 connected to distal support arm 212. Distal support arm 212 extends away from linear member 211 of robotic drive 24 and may be attached to, for example, a frame of robotic drive 24. Device support connection 210 and distal support arm 212 are configured to provide a distal fixed point to support a distal end of a device support (not shown) in a cassette mounted to drive module 206a that is closest to the patient. Device support connection 210 may also be coupled to an introducer sheath hub (not shown).

Each stage 203a-d may be independently actuated to move linearly along rail 204 of linear member 211. Accordingly, each stage 203a-d (and the corresponding drive module 206a-d coupled to the stage 203a-d) may independently move relative to each other and linear member 211. A drive mechanism is used to actuate each stage 203a-d. The drive mechanism includes independent stage translation motors 207a-d coupled to each stage 203a-d and to a stage drive mechanism. The stage drive mechanism may comprise one or more of a lead screw, a rack, a belt, and a chain, for example.

The stage drive mechanism of FIG. 3 is a rack and pinion linear actuator mechanism that includes rack 202 and a separate pinion for each stage 203a-d. Teeth of each pinion (not shown) mesh with teeth of rack 202. To move a stage 203a-d along rail 204, its pinion is rotated in an appropriate direction to travel along rack 202 and push its drive module 206a-d in the direction of travel while rack 202 remains stationary.

In order to reduce a length of linear member 211, offset brackets 208a-d may be configured to create offsets between a stage 203a-d and a drive module 203a-d to reduce a size of gaps between stages 203a-d on linear member 211 when the drive modules are brought together. The length of each drive module 208a-d (and an associated cassette) may limit how close each stage may be brought to an adjacent stage on rail 202.

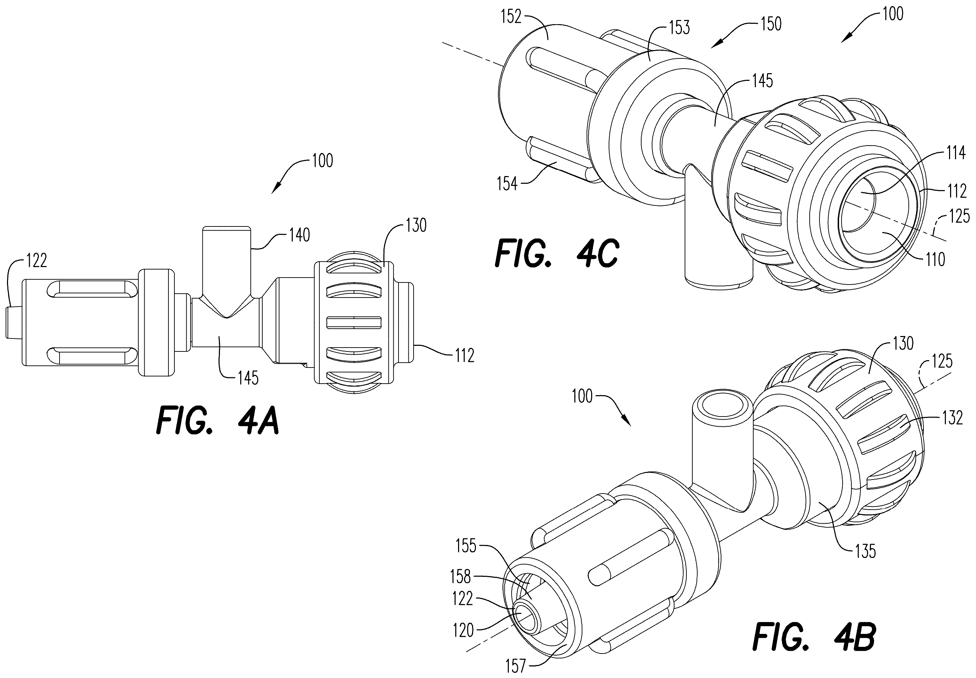

FIGS. 4A-4C illustrate perspective views of rotating hemostasis valve 100. A device according to some embodiments may be coupled to valve 100 in order to facilitate robotic control of an EMD coupled to valve 100. Valve 100 may comprise any rotatable hemostasis valve that is or becomes known. In some embodiments, valve 100 is an "off-the-shelf" valve which is generally available for use by disparate catheter-based interventional systems, including but not limited to manual catheter-based interventional systems (i.e., systems which do not using a robotic drive to manipulate EMDs). Embodiments may be implemented with any off-the-shelf hemostasis valve that is or becomes known.

Valve 100 may comprise a polycarbonate body, but embodiments are not limited thereto. Valve 100 includes proximal port 110 adjacent proximal end 112 and distal port 120 adjacent distal end 122. Valve 100 defines a lumen extending between proximal port 110 and distal port 120 and having longitudinal axis 125. The lumen is in fluid communication with and substantially perpendicular to a lumen of leg 140 at valve portion 145. In other embodiments, leg 140 may be disposed at other angles with respect to leg 140 and/or valve portion 145.

Controllable valve 114 is disposed within proximal port 110. Valve actuator 130 is disposed at proximal end 112 and is rotatable about axis 125 to open and close controllable valve 114. Valve 114 may comprise a material suitable for forming, when closed, a fluid-tight seal around an element (e.g., a guidewire) disposed in proximal port 110. Controllable valve 114 may be controllable to gradually narrow or widen an opening within proximal port 110. Controllable valve 114 may comprise any type of valve and may be actuated (i.e., open and closed) using any suitable mechanism.

Valve 100 further includes rotating luer connector, or rotator, 150 at distal end 122. Rotator 150 includes external surface 152 and internal region 155 presenting male luer interface 158 to which an EMD may be releasably coupled. Luer connectors are known in the art to provide a fluid-tight connection between a catheter and a hemostasis valve. Luer connectors are covered by standards such as ISO 594 (including sections 594-1 and 594-2) and EN 1707.

Rotation of rotator 150 causes rotation about longitudinal axis 125 of a catheter coupled to luer interface 158. This rotation may occur while leg 140 and valve actuator 130 remain substantially still. External surface 152 of rotator includes protrusions 154 which may facilitate manual gripping and rotating of rotator 150.

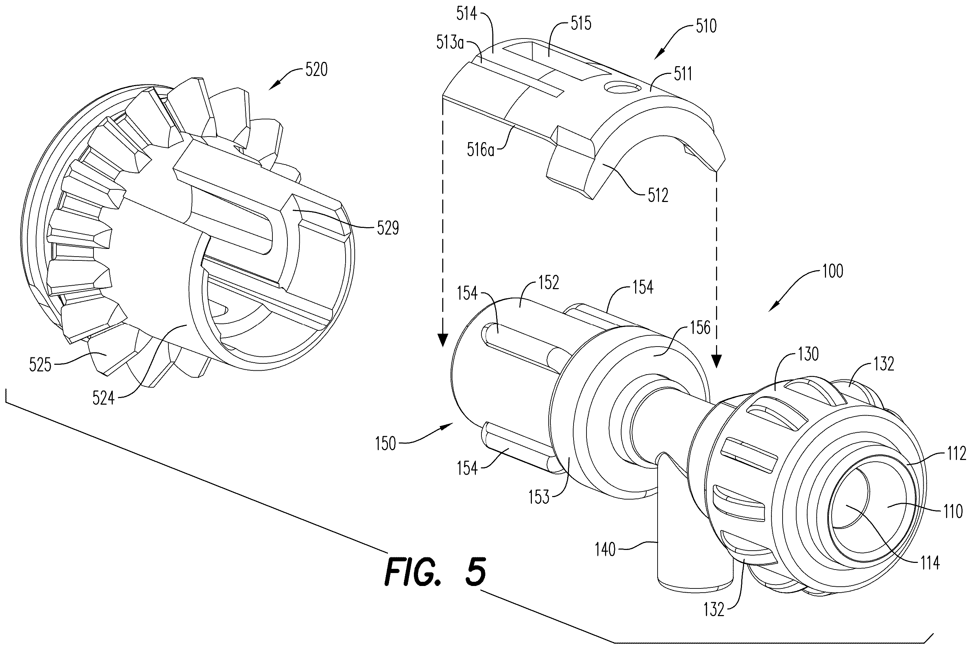

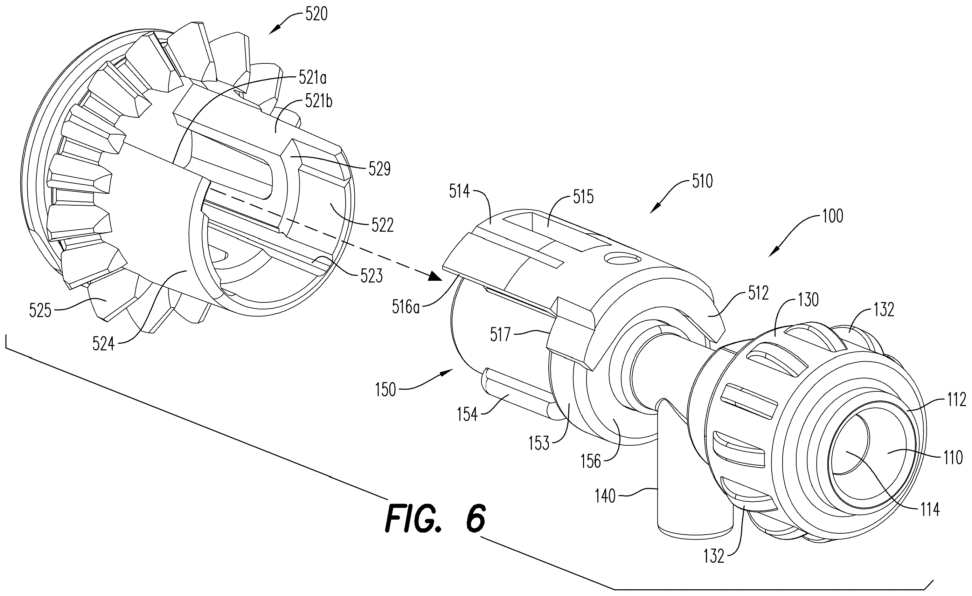

FIGS. 5 and 6 illustrate coupling of a device to a rotator of a hemostasis valve according to some embodiments. The device includes first portion 510 and second portion 520, but embodiments are not limited to devices consisting of only two portions. Portions 510 and 520 may be composed of acetal but are not limited thereto or to a single material. In some embodiments, portions 510 and 520 are composed of different materials.

Portion 510 includes exterior surface 511 and lower lip 512. Exterior surface 511 may be generally curved relative to a horizontal plane and may follow the curvature of external surface 152. Portion 510 includes slots 513a and 513b to impart flexibility to locking portion 514 for reasons which will become evident. Locking portion 514 defines opening 515 for further providing flexibility to portion 514 and for receiving a projection of portion 520 as will be described below.

Portion 520 has a generally cylindrical shape corresponding to curvature of external surface 152 and the generally cylindrical shape of hemostasis valve 100. Portion 520 includes exterior surface 524 and gear 525. The teeth of gear 525 may be integral with exterior surface 524, attached thereto, or otherwise coupled. As will be described below, gear 525 is intended to interface with a drive element of a robotic drive and to be rotated thereby. Embodiments are not limited to a gear as the driven element or to the gear teeth shape, number, diameter or arrangement of the present figures.

As shown in FIGS. 5 and 6, portion 510 is placed onto rotator 150 of valve 100. In the illustrated embodiment, portion 510 is positioned between two protrusions 154 such that surfaces 516a and 516b (not shown in FIGS. 5 and 6) of portion 510 contact the two protrusions 154 along their lengths. An interior (unshown) surface of lower lip 512 contacts side surface 153 and end surface 156 of rotator 150.

To couple portion 520 to portion 510 and to rotator 150, portion 520 is moved onto rotator 150 from distal end 122 toward proximal end 112 of valve 100. This movement causes surfaces 521a and 521b to contact and slide along respective surfaces 516a and 516b. Interior surface 522 of portion 520 defines channels 523 which receive and engage with protrusions 154 as portion 520 is slid toward proximal end 112. Embodiments may provide any other suitable features to engage with protrusions 154. According to some embodiments, external surface 152 of rotator 150 defines, in addition to or instead of protrusions 154, recesses/indentations with which raised features of interior surface 522 engage when portion 520 is coupled to portion 510. The coupling of portion 520 to portion 510 is complete once interior ledge 529 of portion 520 contacts stop 517 of portion 510.

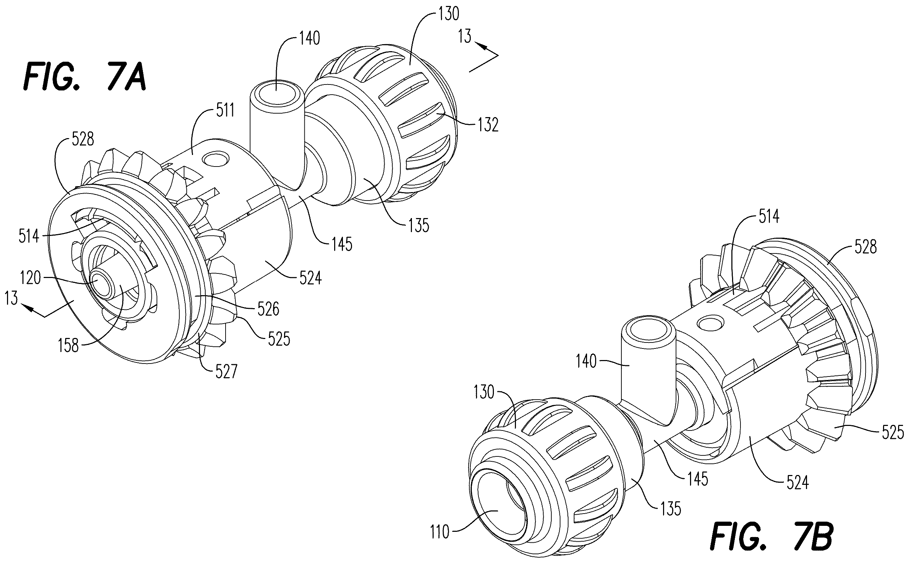

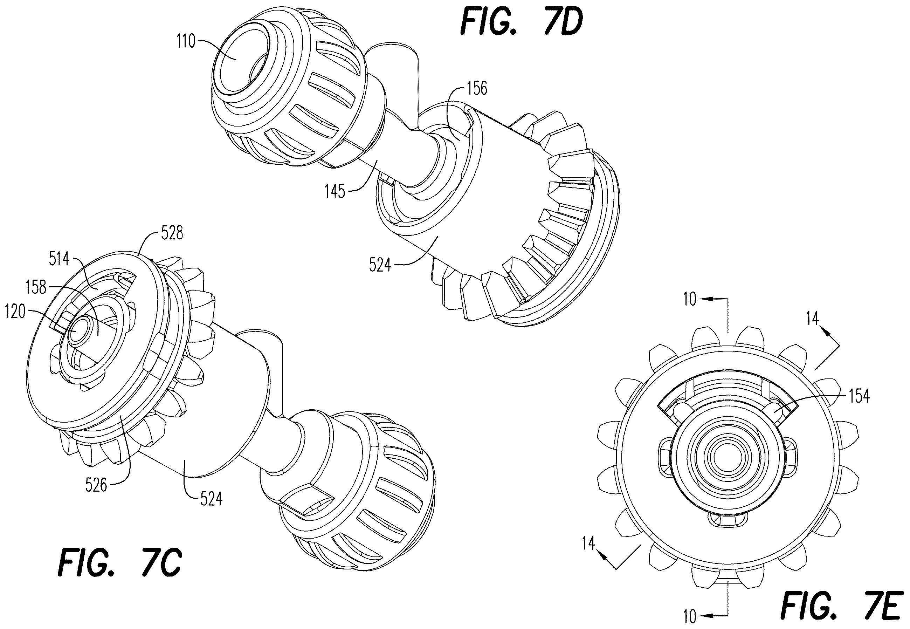

FIGS. 7A - 7D illustrate valve 100 with a device consisting of portions 510 and 520 coupled thereto. The device consisting of portions 510 and 520 does not extend beyond the end of luer connector 158 when portions 510 and 520 are coupled to valve 100. Advantageously, portions 510 and 520 allow use of valve 100 by a robotic drive without increasing the effective length of valve 100.

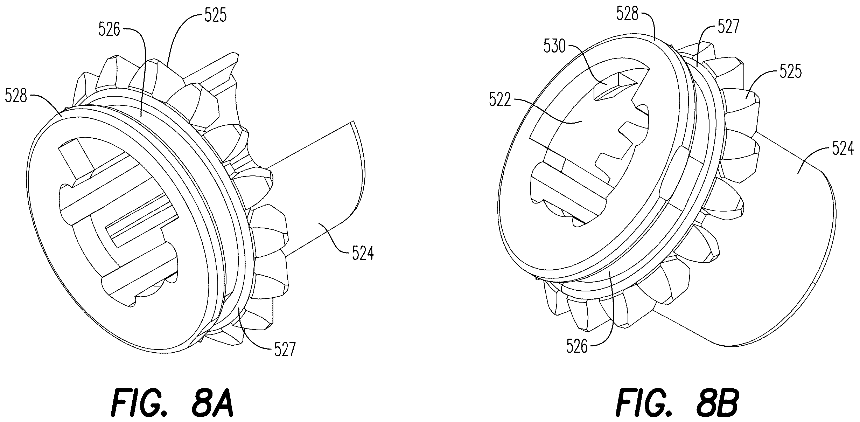

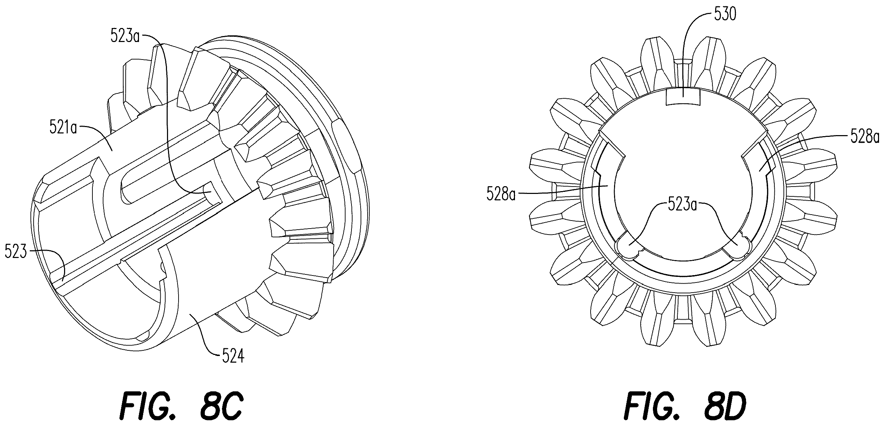

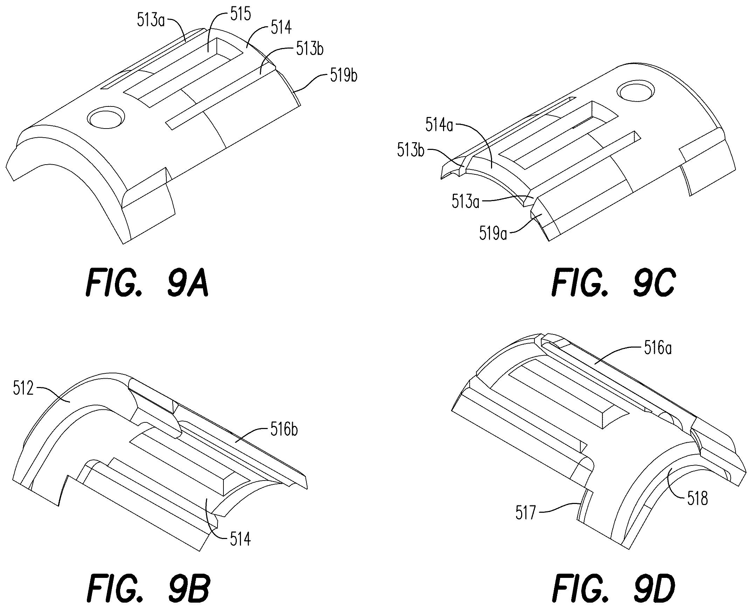

The following description of the system depicted in FIGS. 7A - 7D will also refer to elements of portions 510 and 520 shown in FIGS. 8A - 8D and 9A - 9D. For example, channels 523 are fixedly coupled to (e.g., biased against) protrusions 154 of rotator 150, with channel ends 523a fixedly coupled to ends of protrusions 154. Interior surface 518 of lower lip 512 is fixedly coupled to side surface 153 and end surface 156 of rotator 150. Surfaces 521a and 521b are also biased against surfaces 516a and 516b.

Portion 520 includes projection 530 extending from interior surface 522. Projection 530 is beveled to engage with bevel 514a of flexible portion 514 during the sliding of portion 520 onto portion 510 as described herein. As the sliding continues, projection 530 enters opening 515 of flexible portion and fixedly resides therein while portions 510 and 520 are coupled to rotator 150 as shown in FIGS. 7A- 7E.

Portions 510, portion 520 and rotator 150 are fixedly coupled to one another in view of the above-described coupling of the various surfaces and elements of portions 510 and 520 to one another and to surfaces and elements of rotator 150. While in this arrangement, driving of gear 525 will thereby cause rotation of rotator 150 about axis 125.

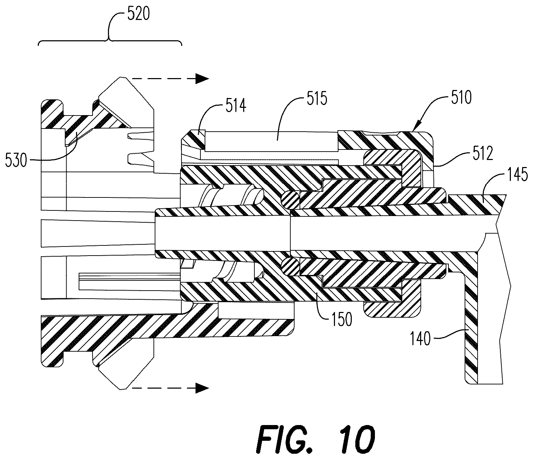

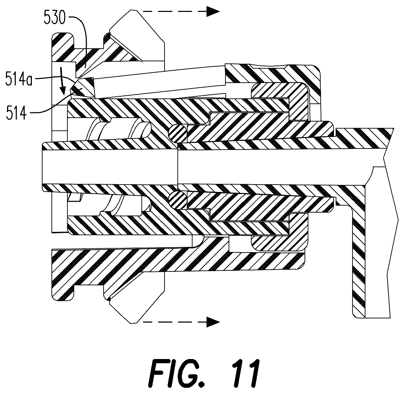

FIGS. 10 - 12 are cross-sectional views of a rotating hemostasis valve during coupling of a device thereto according to some embodiments. FIG. 10 shows first portion 510 coupled to rotator 150 as described above, and portion 520 having slid approximately halfway onto rotator 150. Next, in FIG. 11, portion 520 has slid farther along rotator 150 causing projection 530 to bias against bevel 514a of flexible portion 514. This bias causes flexible portion 514 to flex away from projection 530 as shown.

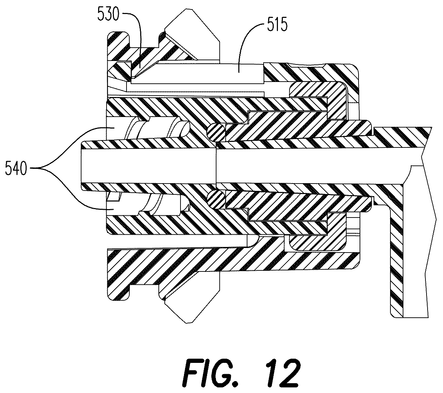

Finally, in FIG. 12, portion 520 has slid further until projection 530 has entered opening 515. Flexible portion 514 no longer flexes away from projection 530 as shown in FIG. 11. Rather, projection 530 fixedly resides within opening 515. In some embodiments, flexible portion 514 may be manually depressed to release projection 530 from opening 515 and allow portion 520 to slide off of portion 510.

Embodiments are not limited to portions 510 and 520 or to the above-described coupling thereof. Embodiments encompass any system to couple a driven element to a rotator of a rotating hemostasis valve. Such systems may utilize adhesive, friction, threads or any other coupling mechanisms.

FIGS. 10 - 12 also show a cross-section of threads 540 of luer connector 158. Threads 540 may be used to facilitate attachment of an EMD having a luer to valve 100. Embodiments are not limited to a threaded luer connector.

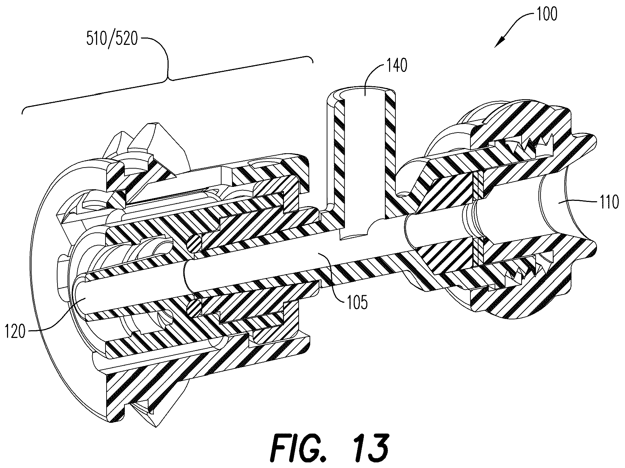

FIG. 13 is a cross-sectional perspective view of valve 100, portion 510 and portion 520 according to some embodiments. Lumen 105 extends between proximal port 110 and distal port 120. Although leg 140 and its lumen are disposed perpendicular to lumen 105, embodiments are not limited thereto.

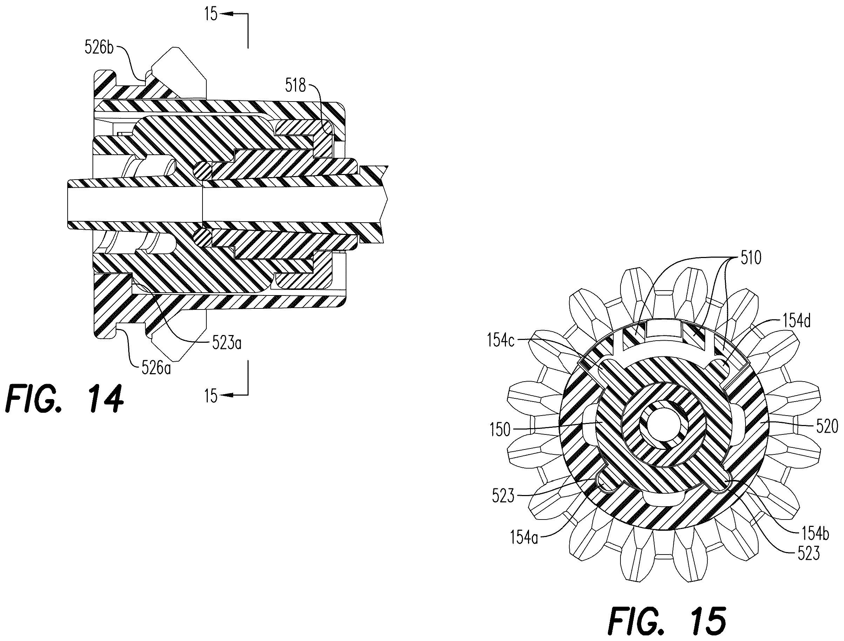

FIGS. 14 and 15 are cross-sectional views of portions 510 and 520 coupled to a rotating hemostasis valve according to some embodiments. Channels 523 of portion 520 are fixedly coupled to protrusions 154a, 154b of rotator 150.

Exterior surface 524 of portion 520 includes groove 526 defined by inner lip 527 and outer lip 528. As will be described below, elements of a robotic drive may interface with groove 526, inner lip 527 and outer lip 528 to bias (and thereby move) portion 520, portion 510 and valve 100 longitudinally.

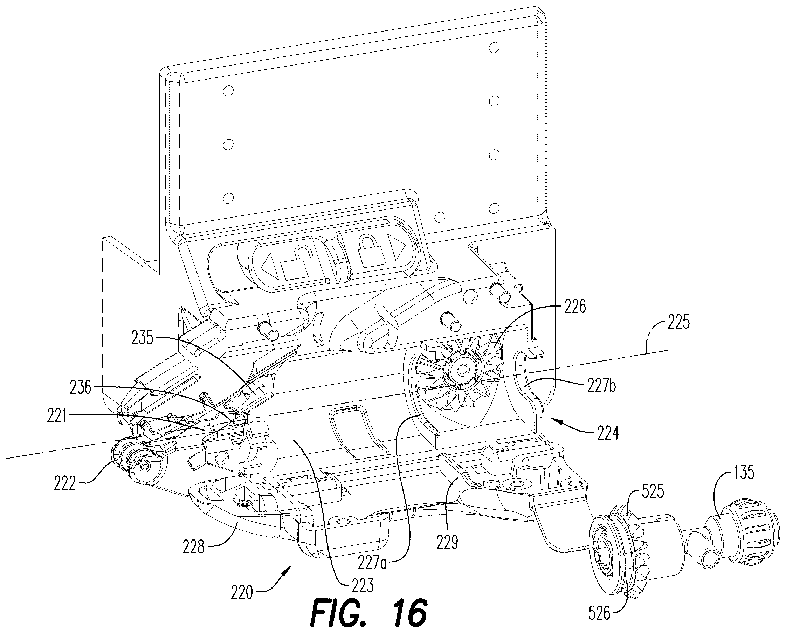

A cassette may be mounted to each drive module 206a-d of robotic drive 24. FIG. 16 is a view of cassette 220 in accordance with some embodiments. Cassette 220 includes features (not shown) that enable cassette 220 to be mounted to a drive module of a robotic drive. Cassette 220 includes distal end 222, cradle 223, proximal end 224 and longitudinal device axis 225. Cassette 220 may comprise any configuration of features suitable to support an EMD and impart linear and rotational motion to the EMD.

Cradle 223 is configured to receive an EMD such as a catheter, guidewire, stent, stent retriever, microwire, microcatheter, etc. Cradle 223 extends longitudinally along longitudinal device axis 225. Cradle may also receive a collet or similar device adapter. Cradle 223 may receive a rotating hemostasis valve and may comprise drive element 226 and support 227b. Mounting cassette 220 onto a drive module 206a-d causes drive element 226 to engage with a coupler 209a-d of the drive module 206a-d. Drive element 226 may comprise a bevel or miter gear in some embodiments. Support 227b has surfaces that are perpendicular or substantially perpendicular to device longitudinal axis.

Support 227a is configured to receive groove 526 of portion 520 and support 227b is configured to receive portion 135 of valve 100. Cover 228 is shown in an open position and includes rib 229. When cover 228 is closed, rib 229 is disposed in groove 526.

Cassette 220 may also include at least one channel 221 in which a device support (not shown) is positioned, where each device support may be a flexible tube with a lengthwise slit and is used to prevent an EMD from buckling. In an embodiment, the system comprises multiple drive modules having at least one cassette attached thereto. A portion of the device support is positioned within the channel of each cassette and a portion of the device support is capable of extending between the cassettes to support at least one EMD positioned in the device support. In one embodiment, a distal end of the device support may be connected to the proximal end of a cassette.

Channel 221 may be angled, curved or offset with respect to the longitudinal device axis 225. Guide 235 and splitter 236 may be positioned on opposite sides of a path of a device support as it is moved into and out of channel 221. A connector holds open an end of the device support allowing it to pass over splitter 236 which opens the slit on the device support and allows the device support to envelope any EMDs passing through cradle 223. Each device support is fixed or constrained at both ends so that the device support may be kept in tension and to limit the amount of displacement over which it may buckle.

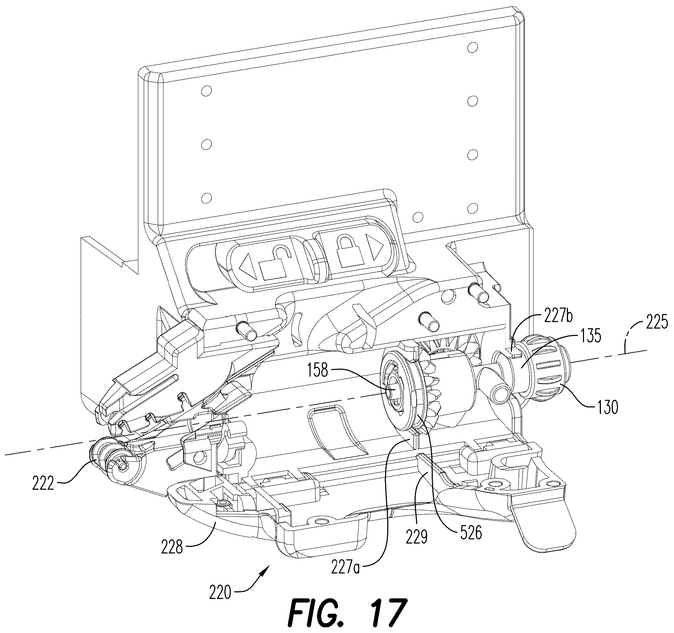

FIG. 17 illustrates valve 100 and portions 510/520 loaded into cassette 220. Specifically, support 227a supports groove 526 of portion 520 and support 227b supports portion 135. Drive element 226 is positioned to engage with gear 525 when groove 526 is supported by support 227a and portion 135 is supported by support 227b. Therefore, operating a drive motor of the drive module 206a-d to which cassette 220 is mounted causes rotation of corresponding coupler 209a-d, rotation of drive element 226 of cassette 220, rotation of gear 525, rotation of rotator 150 of valve 100, and rotation of an EMD which may be attached to luer connector 158.

The dimensions of support 227a may allow groove 526 to rotate somewhat freely while support 227a supports groove 526. In contrast, the dimensions of support 227b may cause portion 135 to sit firmly within (e.g., snap into) support 227b during rotation of rotator 150 or valve actuator 130, and during longitudinal movement of cassette 220. In some embodiments, drive element 226 is located a distance from distal end 222 which allows an operator to attach and remove an EMD to and from rotating luer connector 158 while valve 100 is in the position depicted in FIG. 17.

As described above, a drive module 206a-d may be moved in a longitudinal direction. Movement of a drive module 206a-d to which cassette 220 is coupled in the distal direction of axis 225 biases support 227a and rib 229 against surface 526a of portion 520 and biases interior surface 518 of lower lip 512 against end surface 156 of rotator 150, thereby causing valve 100 and an EMD attached thereto to move in the distal direction. Moreover, movement of the drive module 206a-d in the proximal direction of axis 225 biases support 227a and rib 229 against surface 526b of portion 520 and biases channel ends 523a of portion 520 against the ends of ribs 154a and 154b of rotator 150, thereby causing valve 100 and an EMD attached thereto to move in the proximal direction.

During a procedure, cassette 220 may be moved in the proximal direction of axis 225 to withdraw an EMD attached to luer connector 158. This movement results in a force which pulls portion 520 in the distal direction (via support 227a and rib 229 against outer lip 528) and a force which pulls portion 510 in the proximal direction (via end surface 156 of rotator 150 against interior surface 518 of lower lip 512). By virtue of the locking mechanism of projection 530 and locking portion 514, embodiments are capable of experiencing these opposite forces without resulting in separation of portion 510 from portion 520.

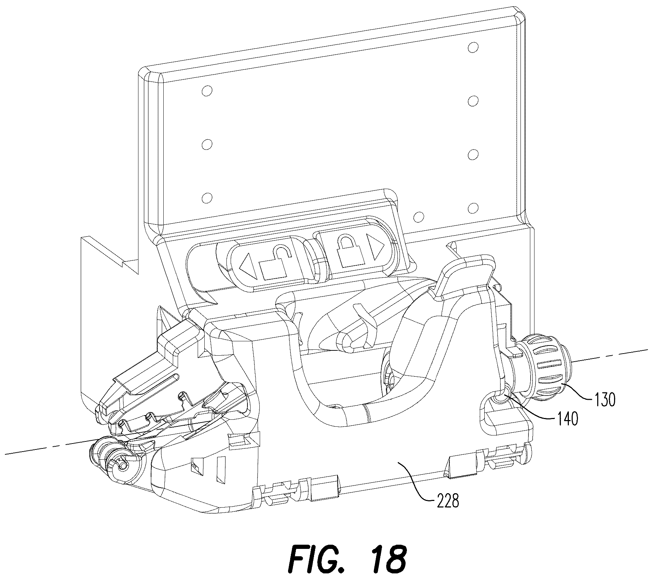

FIG. 18 shows cover 228 is in a closed position. Leg 140 remains exposed when cover 228 is closed, allowing connection of a tube (not shown) thereto facilitate the flow of a fluid (e.g., saline) to and from the valve 100 and an EMD attached thereto. Valve actuator 130 also remains exposed, allowing an operator to rotate actuator 130 to open valve 114, insert an EMD into valve 114 and into an EMD (e.g., a catheter) attached to luer connector 158, and rotate actuator 130 to close valve 114 around the inserted EMD.

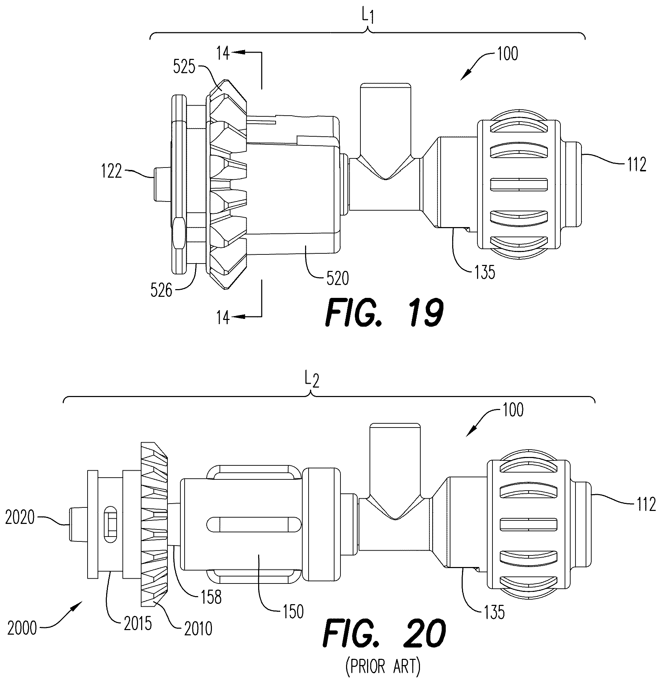

FIG. 19 is a side view of rotating hemostasis valve 100 and a device coupled thereto according to some embodiments, and FIG. 20 is a side view of a prior art system using the same rotating hemostasis valve 100.

The FIG. 20 system includes gear assembly 2000 coupled to rotating luer connector 158 of valve 100. Gear assembly 2000 includes gear 2010, groove 2015 and luer connector 2020. Gear 2010 is intended to be driven by a drive element of a robotic drive cassette, resulting in rotation of rotator 150. Groove 2015 is intended to interface with a support of the cassette as described with respect to groove 526 above. A catheter or other EMD is coupled to luer connector 2020 during operation and rotates with rotation of rotator 150.

During operation, the connection between gear 2010 and connector 158 may separate if the torque applied to gear 2010 is greater than the torque applied to the connection. In contrast, the connection between portion 520 including gear 525 and rotator 150 can tolerate significantly higher torques applied to gear 525 for transmission to rotator 150.

Length L₂ from a tip of connector 2020 to proximal end 112 of the FIG. 20 system is greater than length L₁ from distal end 122 to proximal end 112 of the FIG. 19 system. Accordingly, for a given EMD, the FIG. 19 system provides greater EMD working length than the FIG. 20 system. Also, features of a cassette which support portion 135 and groove 2015 of the FIG. 20 system are necessarily farther apart from one another than cassette features which support groove 526 and portion 135 of the FIG. 19 system, potentially reducing the space available inside the cassette for manually attaching an EMD to a luer connector.

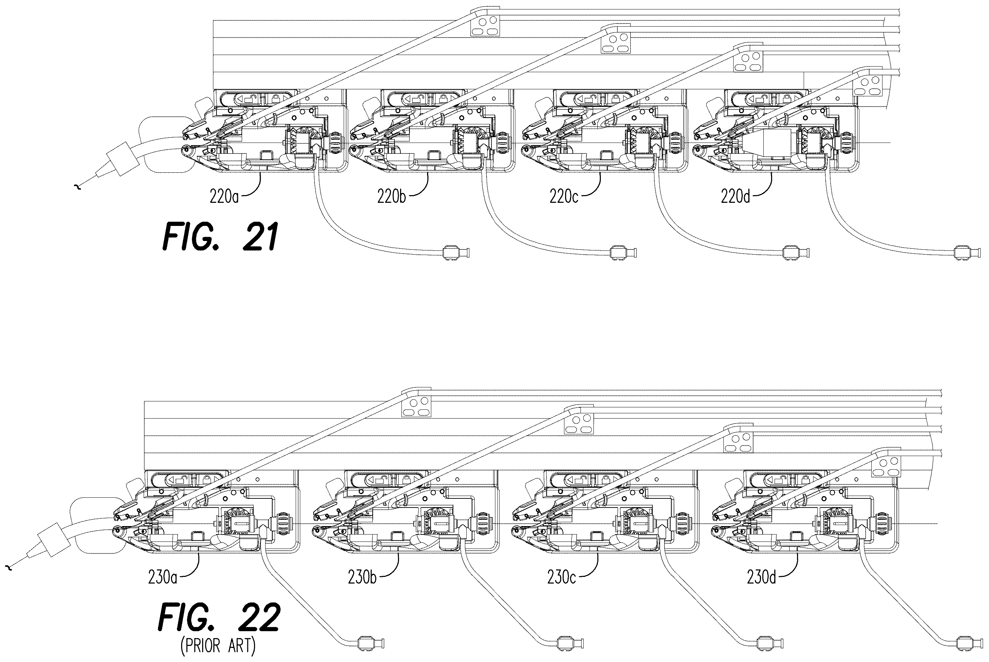

FIG. 21 is a side view of a robotic drive including cassettes 220a-d supporting rotating hemostasis valves according to some embodiments. Each cassette 220a-d includes the above-described system consisting of valve 100 and portions 510/520 and a corresponding EMD attached thereto. Embodiments are not limited to four cassettes or to the use of valve 100 and portions 510/520 in each of cassettes 220a-d. In some embodiments, valve 100 and portions 510/520 are present in less than all of the available cassettes.

FIG. 22 is a side view of a robotic drive including cassettes 230a-d each supporting the rotating hemostasis valve of FIG. 20. The width of each cassette 230a-d is greater than the width of each cassette 220a-d to accommodate the distance between groove 2015 and portion 135. This increased width reduces the effective working length of the EMDs loaded into cassettes 230a-d.

## Claims

1. A device comprising:
an exterior surface comprising a driven element to interface with a drive element of a robotic drive; and
an interior surface comprising features to fixedly couple to a rotator of a rotating hemostasis valve such that driving the driven element with the drive element causes motion of the rotator.

2. The device of Claim 1, wherein the motion comprises rotation of the rotator, and/or wherein the motion comprises longitudinal movement of the rotator.

3. The device of Claim 1 or 2, wherein the device does not extend beyond an end of a male luer connector of the rotating hemostasis valve when the features of the interior surface are fixedly coupled to the rotator.

4. The device of one of Claims 1 to 3, wherein the device comprises a first portion and a second portion, the first portion and the second portion comprising one or more features to fixedly couple the first portion and the second portion when the features of the interior surface are fixedly coupled to the rotator.

5. The device of Claim 4, wherein the first portion comprises the driven element and a first surface to interface with a first end of the rotator, wherein the first portion particularly comprises a second surface to interface with an end of a protrusion extending from a surface of the rotator.

6. The device of one of Claims 4 to 5, wherein the first portion comprises a projection extending from the interior surface, and
wherein the second portion comprises a flexible portion defining an opening in which the projection fixedly resides when the features of the interior surface are fixedly coupled to the rotator,
wherein the projection particularly biases the flexible portion to flex away from the projection as the first portion is slid over the second portion.

7. The device of Claim 6, wherein the first portion comprises the features to fixedly couple to the rotator.

8. The device of one of Claims 1 to 7, wherein the driven element comprises a gear to rotate the rotator and a feature to interface with the robotic drive to bias the device longitudinally.

9. The device of one of Claims 1 to 8, comprising a first surface to interface with a first end of the rotator and a second surface to interface with an end of a protrusion extending from a surface of the rotator.

10. A device comprising:
an exterior surface comprising a driven element to interface with a drive element of a robotic drive; and
an interior surface comprising first features to couple to second features of a rotator of a rotating hemostasis valve such that driving the driven element with the drive element causes movement of the rotator,
wherein the device does not extend beyond an end of a male luer connector of the rotating hemostasis valve when the first features of the interior surface are coupled to the rotator.

11. The device of Claim 10, wherein the device comprises a first portion and a second portion, each of the first portion and the second portion comprising one or more features to fixedly couple the first portion and the second portion when the first features of the interior surface are coupled to the second features of the rotator, wherein the first portion particularly comprises the driven element and a first surface to interface with a first end of the rotator, and
wherein the first portion particularly comprises a second surface to interface with an end of one of the second features of the rotator.

12. The device of Claim 11, wherein the first portion comprises a projection extending from the interior surface, and
wherein the second portion comprises a flexible portion defining an opening in which the projection fixedly resides when the first features of the interior surface are coupled to the second features of the rotator.

13. The device of Claim 12, wherein the projection biases the flexible portion to flex away from the projection as the first portion is slid over the second portion.

14. A method comprising:
coupling a first portion of a device to a rotator of a rotating hemostasis valve;
coupling a second portion of the device to the first portion and to the rotator of the rotating hemostasis valve; and
inserting the rotating hemostasis valve into a robotic drive to couple a driven element of the second portion to a drive element of the robotic drive; and
operating the drive element to drive the driven element,
wherein the driving of the driven element causes movement of the rotator.

15. The method of Claim 14, wherein coupling the second portion of the device to the first portion comprises sliding the second portion over the first portion to bias a projection extending from the second portion against a flexible portion of the first portion until the projection fixedly resides within an opening of the flexible portion.
